(19) Europäisches Patentamt
European Patent Office
Office européen des brevets

(11) Publication number: **0 113 988 B1**

# (12) EUROPEAN PATENT SPECIFICATION

(45) Date of publication of patent specification: **22.06.88**

(51) Int. Cl.$^4$: **A 23 L 1/221**, C 11 B 9/02, A 23 G 3/00

(21) Application number: **83307701.9**

(22) Date of filing: **19.12.83**

## (54) A process for preparing a reduced-menthofuran-content peppermint oil.

(30) Priority: **20.12.82 US 450870**

(43) Date of publication of application: **25.07.84 Bulletin 84/30**

(45) Publication of the grant of the patent: **22.06.88 Bulletin 88/25**

(84) Designated Contracting States: **BE CH DE FR GB LI NL**

(56) References cited:
**US-A-3 083 105**
**JOURNAL OF THE AMERICAN CHEMICAL SOCIETY, vol. 70, February 1948, pages 621-622; P.Z.BEDOUKIAN: "Occurrence of menthofuran in oil of peppermint (Mentha piperita vulgaris S.)"**
**JOURNAL OF THE AMERICAN CHEMICAL SOCIETY, vol. 72, November 1950, pages 5313-5314; R.H.EASTMAN: "The isolation of menthofuran from american peppermint oil"**
**FURIA et al.: "FENAROLI'S HANDBOOK OF FLAVOR INGREDIENTS", 1971, pages 195-196, The Chemical Rubber Co., Cleveland, Ohio, US;**

(73) Proprietor: **WARNER-LAMBERT COMPANY**
**201 Tabor Road**
**Morris Plains New Jersey 07950 (US)**

(72) Inventor: **Blackwell, Bernie**
**226-06 139th Avenue**
**Laurelton New York 11413 (US)**
Inventor: **Netherwood, Shelley**
**Raintree Apartments No. 2814 650 Tamarac Avenue**
**Brea California 92621 (US)**
Inventor: **Piccolo, Dominic Joseph**
**292 19th Street**
**Brooklyn New York 11215 (US)**

(74) Representative: **Jones, Michael Raymond et al**
**HASELTINE LAKE & CO. Hazlitt House 28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 113 988 B1

Courier Press, Leamington Spa, England.

**Description**

This invention relates to a process for preparing a reduced-menthofuran-content peppermint oil.

Peppermint oil is a well known flavouring agent derived from the leaves and flowering tops of the plant Mentha poperita L. The plants' habitat is quite diverse and it has been found in parts of Asia, Europe and North America.

The peppermint oil constituents vary depending upon the source of the plant although several main constituents are usually present. In general, peppermint oil contains varying amounts of alpha-pinene, beta-pinene, limonene, 1,8-cineole, menthone, menthofuran, isomenthol, menthyl acetate, neomenthol and menthol as well as various amounts of other constituents.

One disadvantage associated with the use of peppermint oil in the past has been its degradation during storage, resulting in a change in odour and flavour properties. Several studies have been undertaken to identify the aging characteristics of peppermint oil.

One study is described by R. H. Reitsema, et al. in *Oxidation of Peppermint Oil,* Industrial and Engineering Chemistry, Vol 44, page 176, January 1952, wherein three reactions which contribute to the aging process are described: the first reaction is the isomerization of terpenes which is described therein as having only minor importance; the second reaction involves the polymerization of the oil constituents which is noted as increasing the oils' molecular weight and the viscosity of the resulting product; and thirdly, the most important reaction, the oxidation of the peppermint oil which causes aging of the oil from a flavour standpoint. Specific components involved in the oxidation-aging process include the pinenes, limonene and menthofuran.

Various attempts have been made to inhibit the oxidation process to prevent the formation of oxidative by-products commonly referred to as "off-note" material. One conventional technique involves the use of anti-oxidants in the peppermint oil in an effort to inhibit the oxidation reactions from occurring. The use of such anti-oxidants, however, has not been successful since the anti-oxidants are either not effective in suppressing the oxidation reaction over prolonged storage periods or they have resulted in the formation of "off-note" peppermint oils detracting from the flavouring agent properties. These processes, at best, have merely attempted to suppress the formation of oxidation process by-products without removing from the oil the oxidation constituents themselves.

Another study aimed at attempting to overcome the oxidation phenomenon is disclosed by Guenther in *The Essential Oils,* Vol III (1949), page 619. This reference discloses use of a fractional distillation technique to separate the peppermint oil flavour constituents from menthofuran. This technique was unsuccessful, however, since the menthofuran was codistilled with menthone, a major flavouring component of peppermint oil, this rendering the recovered product unsuitable for use as a flavouring agent.

A process which selectively removes one of the primary "off-note" oxidative by-product contributors, namely menthofuran, has been unexpectedly discovered.

According to one aspect of the present invention, there is provided a process for preparing a reduced-menthofuran-content peppermint oil, which process comprises:

(a) reacting, at a temperature of from 0°C to 80°C, a starting peppermint oil with an amount of maleic anhydride in the range from 0.5% to 15% by weight, based on the weight of starting peppermint oil, to selectively form a menthofuran-maleic anhydride adduct without otherwise affecting the flavouring properties of the remaining peppermint oil constituents;

(b) contacting the reaction mixture of step (a) with an aqueous basic solution to neutralize any free maleic acid present; and

(c) by subjecting the product of step (b) to vacuum distillation of steam distillation, recovering a peppermint oil having menthofuran content of from 0.8% to 1.4%, based on the weight of peppermint oil.

The structure of menthofuran is chemically known as tetrahydro-4,5,6,7-dimethyl-3,5-coumarone and is routinely found in peppermint oil in amounts of up to 15% and generally in amounts of from 1 to 10% based on the weight of the peppermint oil.

The addition of maleic anhydride to peppermint oil to identify the existence and presence of menthofuran was proposed by P. Bedoukian, *Journal of American Chemical Society,* Vol 70, page 621, 1948 and R. Eastman, *Journal of American Chemical Society,* Vol 72, page 5313, 1950. Both Bedoukian and Eastman disclose the formation of a crystalline material, theorised to be menthofuran-maleic anhydride adduct, upon the addition of maleic anhydride to peppermint oil in the presence of a benzene solvent. The impure peppermint oil comprising the benzene solvent was discarded and the menthofuran chemically identified. Each of these techniques employed excess maleic anhydride and benzene as a solvent to stabilize the menthofuran-maleic anhydride adduct.

In contrast to these techniques, the present invention employs the above-mentioned basic reaction in an unexpected manner to purify peppermint oil.

It has been found that this basic reaction can be used to prepare a reduced-menthofuran-content peppermint oil which has a superior resistance, compared to the starting peppermint oil, to the formation of oxidative "off-note" materials, or musty odour associated with the oxidation by-products.

It has been found that if a sufficient amount of maleic anhydride is added to the starting peppermint oil a menthofuran-maleic anhydride adduct is formed which does not concurrently enable formation of

secondary by-products rendering the oil unusable. A proposed scheme for the formation of the adduct is illustrated as follows:

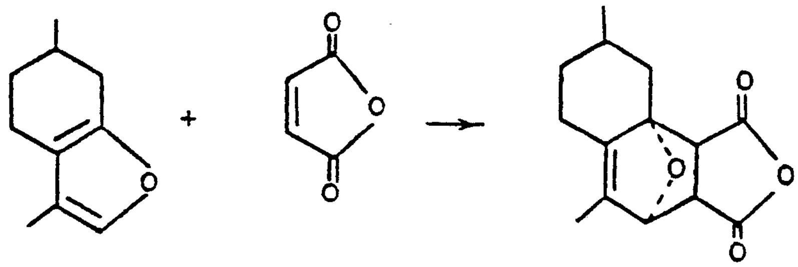

It appears from the stoichiometry of the reaction that the adduct may result from a Diels-Alder type addition and that the 1,4 conjugated diene system in the menthofuran attaches to the carbon-carbon double bond between the two carbonyl groups in maleic anhydride. Once the adduct is removed from the starting peppermint oil, the resulting oil has the normal constituent profile and balance of the starting oil but with a reduced-menthofuran-content.

The amount of maleic anhydride employed in this process is, preferably, sufficient to complex with a portion of the menthofuran whilst enabling a portion of the menthofuran to be retained in low amounts in the reduced-menthofuran-content peppermint oil. In one embodiment of the present invention an acceptable amount of maleic anhydride has been found to be the stoichiometric amount of maleic anhydride needed to react with the menthofuran present in the starting peppermint oil. In another embodiment the acceptable amount of maleic anhydride has been found to be less than the stoichiometric amount needed to react with the menthofuran present in the starting peppermint oil. When excess amounts of maleic anhydride are employed, removal of all of the menthofuran from the peppermint oil is achieved. The resulting peppermint oil product, however, has a modified appearance and noticeable "off-note" flavour and package aroma. Accordingly, the process of the present invention is preferably performed in a manner which removes only a portion of the menthofuran whilst still enhancing the stability of the peppermint oil.

The reduced-menthofuran-content peppemint oil of the present invention has a menthofuran content, after treatment, in the range from 0.8 to 1.4%, and preferably in the range from 0.8% to 1%, based on the weight of reduced-menthofuran-content peppermint oil. This amount of menthofuran is achieved by using in the range from 0.5% to 15% by weight maleic anhydride by weight of starting peppermint oil. It should be recognized that the exact amount of maleic anhydride employed will vary depending upon the menthofuran content of the starting oil and the degree of reduction in content of menthofuran desired.

It has been found that use of stoichiometric amounts of maleic anhydride based on the menthofuran content of the starting peppermint oil results in lowering the menthofuran content to acceptable levels without completely complexing all the maleic anhydride to form maleic anhydride-menthofuran adduct. This is achieved by the formation, in the peppermint oil, of free maleic acid resulting from the hydrolysis reaction of maleic anhydride and water; water being inherently present in the peppermint oil.

In a preferred embodiment of the invention, a starting peppermint oil containing menthofuran is admixed with the maleic anhydride and stirred to solubilize the maleic anhydride. The reaction between the menthofuran and the maleic anhydride commences almost instantaneously resulting in an exothermic reaction.

The temperature of the reaction admixture is maintained in the range from 0°C to 80°C and preferably in the range from 25°C to 50°C. Temperatures below 0°C tend to result in the gelation of the oil while temperatures above 80°C may cause accelerated oxidation and polymerization of the oil constituents. It should be recognized that the reaction is exothermic in nature and use of standard means to maintain the reaction temperatures are contemplated to be used.

The reaction time is not critical and will vary depending upon the particular reaction temperature employed, faster reaction times occur at higher temperatures, and longer reaction times occur at lower temperatures. For commercial reasons the reaction times may vary from a few seconds up to 12 hours and preferably be up to 5 hours although longer times (for example up to 4 weeks) at lower temperatures are useable.

Once the reaction is complete the menthofuran-maleic anhydride adduct is separated from the reduced-menthofuran-content peppermint oil. Since the menthofuran-maleic anhydride adduct is crystalline in peppermint oil at temperatures below 5°C, separation is conveniently performed by lowering the temperature of the admixture to crystallize the menthofuran-maleic anhydride adduct. The resulting reduced-menthofuran-content peppermint oil is then recovered by conventional solid-liquid separation means such as filtration or decantation.

In the present invention, after the starting peppermint oil has been mixed with the maleic anhydride, the reaction mixture is contacted with an aqueous basic solution prior to crystallisation. This enables the neutralisation of any free maleic acid present in the reaction mixture. The presence of free maleic acid may alter the flavour and/or aroma properties of the reduced-menthofuran-content peppermint oil if not removed.

The neutralisation reaction is performed with any basic composition that will react with the maleic acid to form the corresponding water-soluble salt. Suitably exemplary inorganic base materials include sodium carbonate and bicarbonate, potassium carbonate, calcium carbonate, sodium sulphate, potassium sulphate, calcium sulphate, sodium phosphate, potassium phosphate, calcium phosphate, sodium formate, potassium formate, calcium formate, sodium nitrate, potassium nitrate, calcium nitrate and mixtures thereof. The preferred base is sodium carbonate, but any inorganic material may be used which forms the maleate salt and which does not adversely affect the peppermint oil composition to which it is admixed.

The amount of aqueous basic solution added to the reaction mixture may be any amount which results in the neutralisation of the free maleic acid. Typically, use of from 0.5% to 20%, preferably about 10% by weight, based on the weight of the peppermint oil, of basic solution is sufficient.

The neutralisation reaction is performed in a conventional manner by simply mixing the aqueous basic solution with the reaction mixture. Once mixed the resulting oil-water emulsion is permitted to separate into its respective phases, i.e. an oil phase comprising the peppermint oil and the menthofuran-maleic anhydride adduct, and a water phase containing free unreacted base, the maleate salt and any water-soluble impurities associated with the oil. The oil and water phases may then be separated by conventional liquid/liquid separation techniques, for example decantation; the aqueous phase is then discarded and the oil phase is processed to remove the menthofuran-maleic anhydride by the crystallisation technique described above.

In another embodiment, the reduced-menthofuran-content peppermint oil is recovered from the reaction mixture comprising the peppermint oil and the menthofuran-maleic anhydride adduct by vacuum distillation or steam distillation. The distillation operation must be performed in a manner to prevent oxidation and polymerization of the peppermint oil whilst enabling separation of the reduced-menthofuran-content peppermint oil from the adduct.

Suitably exemplary distillation methods include vacuum distillation and steam distillation methods. Use of standard boiling distillation techniques are not acceptable and result in degradation of the peppermint oil. The use of vacuum distillation or steam distillation methods enables the selective removal of the unreacted peppermint oil fractions based on their respective boiling points. As in conventional steam and vacuum distillations, separation is achieved by the condensation of the vapour removed from the distillation device.

The temperature employed during the distillation will vary widely depending upon the vacuum pressure employed to fractionally separate the peppermint oil fractions from the adduct. The temperatures used represent the boiling point of the constituents at the particular pressure employed.

As in conventional vacuum distillation techniques a gas may be employed during the reaction to purge the contents of particular fractions to enhance reaction speed and efficiency. Illustrative gases include any gas that is non-reactive with the peppermint oil constitutents and may be, for example, inert gases, such as argon and helium and well as nitrogen, carbon dioxide, and other suitable gases and mixtures thereof.

Because the boiling point of the menthofuran-maleic anhydride adduct is closely related to the higher molecular weight constituents of the peppermint oil, distillation is preferably performed until most, but not all, of the peppermint oil has been removed. In this regard, it is advantageous to withdraw the peppermint oil fractions until a residue remains in the distillation flask which residue contains the adduct and a minor amount of residual unremoved peppermint oil. The residue in the distillation flask is conveniently discarded. The peppermint oil fractions, which contain a low level of unreacted menthofuran, are then recovered and combined to form the reduced-menthofuran-content peppermint oil of the present invention.

According to another aspect of the present invention, there is provided a peppermint oil composition containing the reduced-menthofuran-content peppermint oil, which composition may be stored, or used directly as a flavouring agent by blending with foods, chewing gum, confectionery, pharmaceuticals, beverages, tobacco and proprietary products such as toothpaste and mouthwash using conventional means and in amounts sufficient to provide the desired flavouring power. Acceptable amounts will vary from about 0.01% to about 5.0% by weight flavouring agent based on the weight of the final product.

According to a further aspect of the present invention, there is provided a method of flavouring foods, chewing gum, confectionery, pharmaceuticals, beverages, tobacco and proprietary products, which method comprises adding thereto an effective amount of reduced-menthofuran-content peppermint oil prepared by a process according to the first aspect of the present invention.

According to a still further aspect of the present invention there are provided foods, chewing gum, confectionery, pharmaceuticals, beverages, tobacco and proprietary products whenever flavoured by a reduced-menthofuran-content peppermint oil prepared by a process according to the present invention.

## Example 1

This Example demonstrates the selective removal of menthofuran from peppermint oil.

Five hundred grams of Midwest peppermint oil (A.M. Todd Company, Kalamazoo, Michigan) containing 2.8% menthofuran were mixed with 10% by weight (50 gm) maleic anhydride in a flask. The mixture was mechanically stirred for a total of 30 minutes at 28°C during which time a rise in temperature to 50°C was noted. The solution was then neutralised by washing with 100 grams of 10% $Na_2CO_3$ solution.

The lower hydrous layer, which contained $Na_2CO_3$, sodium maleate, and water soluble impurities from the peppermint oil, was separated and discarded.

The reduced-menthofuran-content peppermint oil was recovered by distillation under vacuum using a nitrogen purging atmosphere. The distillation unit consisted of a boiling flask, two fractionation columns packed with Berl Saddles, two cold water condensers, a nitrogen embulator, and a graduated vacuum collector. The distillation temperature was maintained between 24°C and 86°C in the pot with a vapour temperature around 57°C.

The yield of recovery product was 461.0 gms. The menthofuran content in the recovered reduced-menthofuran-content peppermint oil was 0.80% determined by capillary gas chromatography. A residue of 16.10 grams (3.00%) which contained some peppermint oil residue and the menthofuran-maleic anhydride adduct, remained in the distillation flask.

Example 2

This Example compares the menthofuran content of a normal peppermint oil with that of a reduced-menthofuran-content peppermint oil produced in accordance with the present invention.

A gas chromatographic analysis of peppermint oil, both prior and subsequent to treatment with maleic anhydride, was used. The results are set forth in Figures 1 and 2.

Figure 1 depicts the gas chromatographic analysis of an American Midwest peppermint oil prior to the purification treatment of this invention.

Figure 2 depicts the gas chromatographic analysis of the same peppermint oil shown in Figure 1 after the purification procedure of Example 1.

The analysis was performed, using a Hewlett Packard Model 5840—A device, by conventional gas-liquid chromatography techniques using a 275°C injection temperature to maintain all of the sample components in the vapour state. The column used a 30 meter glass column containing Carbowax 20M glycols (product of Union Carbide Corporation).

The Figures graphically show the individual components of peppermint oil as they are removed from the column. The results indicate that maleic anhydride selectively reacts with menthofuran without affecting the remaining components of the peppermint oil. This selective reaction enables a reduction in the menthofuran content to be easily achieved without affecting the flavouring agent properties of the peppermint oil associated with the remaining constituents.

**Claims**

1. A process for preparing a reduced-menthofuran-content peppermint oil, which process comprises:

(a) reacting, at a temperature of from 0°C to 80°C, a starting peppermint oil with an amount of maleic anhydride in the range from 0.5% to 15% by weight, based on the weight of starting peppermint oil, to selectively form a menthofuran-maleic anhydride adduct without otherwise affecting the flavouring properties of the remaining peppermint oil constituents;

(b) contacting the reaction mixture of step (a) with an aqueous basic solution to neutralize any free maleic acid present; and

(c) by subjecting the product of step (b) to vacuum distillation or steam distillation, recovering a peppermint oil having menthofuran content of from 0.8% to 1.4%, based on the weight of peppermint oil.

2. A process according to claim 1, wherein the menthofuran content in the recovered peppermint oil is in the range from 0.8% to 1%, by weight, based on the weight of recovered peppermint oil.

3. A process according to claim 1 or 2, wherein the amount of maleic anhydride is the stoichiometric amount needed to react with the menthofuran present in the starting peppermint oil.

4. A process according to claim 1 or 2, wherein the amount of maleic anhydride is less than the stoichiometric amount needed to react with the menthofuran present in starting peppermint oil.

5. A process according to any of claims 1 to 4, wherein step (b) comprises mixing with the reaction mixture an aqueous basic solution to form two phases, which are preferably separated, namely an oil phase comprising the peppermint oil and menthofuran-maleic anhydride adduct, and an aqueous phase comprising free base, a maleate salt and water-soluble impurities.

6. A process according to any preceding claim, wherein the reaction mixture comprising the menthofuran-maleic anhydride adduct is separated by cooling the reaction mixture below its reaction temperature to crystallize the menthofuran-maleic anhydride adduct.

7. A method for producing a composition, which method comprises:

(1) preparing a reduced-menthofuran-content peppermint oil by a process which comprises:

(a) reacting, at a temperature of from 0°C to 80°C, a starting peppermint oil with an amount of maleic anhydride in the range from 0.5% to 15% by weight, based on the weight of starting peppermint oil, to selectively form a menthofuran-maleic anhydride adduct without otherwise affecting the flavouring properties of the remaining peppermint oil constituents;

(b) contacting the reaction mixture of step (a) with an aqueous basic solution to neutralize any free maleic acid present; and

(c) by subjecting the product of step (b) to vacuum distillation or steam distillation, recovering a

peppermint oil having menthofuran content of from 0.8% to 1.4%, based on the weight of peppermint oil; and

(2) incorporating, into food, chewing gum, confectionery, a pharmaceutical, a beverage, tobacco or a proprietary product, from 0.01% to 5% by weight, based on the weight of the resulting composition, of said reduced-menthofuran-content peppermint oil.

8. A method of flavouring food, chewing gum, confectionery, a pharmaceutical, a beverage, tobacco or a proprietary product, which method comprises:

(1) preparing a reduced-menthofuran-content peppermint oil by a process which comprises:

(a) reacting, at a temperature of from 0°C to 80°C, a starting peppermint oil with an amount of maleic anhydride in the range from 0.5% to 15% by weight, based on the weight of starting peppermint oil, to selectively form a menthofuran-maleic anhydride adduct without otherwise affecting the flavouring properties of the remaining peppermint oil constituents;

(b) contacting the reaction mixture of step (a) with an aqueous basic solution to neutralize any free maleic acid present; and

(c) by subjecting the product of step (b) to vacuum distillation or steam distillation, recovering a peppermint oil having menthofuran content of from 0.8% to 1.4% based on the weight of peppermint oil; and

(2) adding, to said food, chewing gum, confectionery, pharmaceutical, beverage, tobacco or proprietary product, an effective amount of said reduced-menthofuran-content peppermint oil.

**Patentansprüche**

1. Verfahren zum Herstellen von Pfefferminzöl mit reduziertem Menthofuran-Gehalt, gekennzeichnet, dadurch:

(a) dass ein Ausgangs-Pfefferminzöl, bei einer Temperatur von 0°C bis 80°C, mit einer Menge von auf das Anfangsgewicht des Pfefferminzöls bezogen 0.5% bis 15% Maleinsäure-Anhydrid, reagiert um ein Menthofuran-Maleinsäure-Anhydrid-Addukt zu bilden, ohne die Aromaeigenschaften der verbleibenden Bestandteile des Pfefferminzöls zu beeinflussen;

(b) dass man der Reaktions-Mischung von Schritt (a) eine wässrige, basische Lösung zusetzt um noch vorhandene Maleinsäure zu neutralisieren; und

(c) dass man das Produkt von Schritt (b) vakuum- oder wasserdampfdestilliert, um ein Pfefferminzöl zu gewinnen, das einen auf das Gewicht des Pfefferminzöls bezogenen Menthofuran-Gehalt von 0.8% bis 1.4% aufweist.

2. Verfahren nach Anspruch 1, bei dem der Menthofurangehalt im gewonnen Pfefferminzöl im Bereich von 0.8% bis 1% des Gewichts des gewonnenen Pfefferminzöls liegt.

3. Verfahren nach Anspruch 1 oder 2, bei dem der Anteil des Maleinsäure-Anhydrids der stöchiometrisch notwendigen Menge entspricht, um mit dem im Ausgangs-Pfefferminzöl vorhandenen Menthofuran zu reagieren.

4. Verfahren nach Anspruch 1 oder 2, bei dem die Menge des Maleinsäure-Anhydrids kleiner als die stöchiometrisch notwendige Menge ist, um mit dem im Ausgangs-Pfefferminzöl vohandenen Menthofuran zu reagieren.

5. Verfahren nach einem Ansprüche 1 bis 4, bei dem der Verfahrensschritt (b) das Mischen der Reaktionsmischung mit einer wässrigen basischen Lösung umfasst, um zwei Phasen zu erzeugen, welche vorzugsweise getrennt sind, nämlich eine Oelphase, die Pfefferminzöl und Menthofuran-Maleinsäure-Anhydrid-Addukt enthält und eine wässrige Phase, welche freie Base, ein Salz der maleinsäure und wasserlösliche Verunreinigungen enthält.

6. Verfahren nach einem der vorherigen Ansprüche, bei dem die Reaktionsmischung, die das Menthofuran-Maleinsäure-Anhydrid-Addukt enthaelt durch Abkühlen der Reaktionsmischung unter ihre Reaktionstemperatur gekühlt wird um das Menthofuran-Maleinsäure-Anhydrid-Addukt zu kristallisieren.

7. Verfahren zum Herstellen eines Präparats, welches unfasst:

(1) des Herstellen eines Pfefferminzöls mit reduziertem Menthofuran-Gehalt nach einem Verfahren das umfasst;

(a) das Reagiern eines Ausgangs-Pfefferminzöls, bei einer Temperatur von 0°C bis 80°C, mit einer Menge von auf das Anfangsgewicht des Pfefferminzöls bezogen 0.5% bis 15% Maleinsäure-Anhydrid, um ein Menthofuran-Maleinsäure-Anhydrid-Addukt zu bilden, ohne die Aromaeigenschaften der verbleibenden Bestandteile des Pfefferminzöls zu beeinflussen;

(b) dass man der Reaktions-Mischung von Schritt (a) eine wässrige, basische Lösung zusetzt um noch vorhandene Maleinsäure zu neutralisieren; und

(c) dass man das Produkt von Schritt (b) vakuum-oder wasserdampfdestilliert, um ein Pfefferminzöl zu gewinnen, das einen auf das Gewicht des Pfefferminzöls bezogenen Menthofuran-Gehalt von 0.8% bis 1.4% aufweist, und

(2) dass man, bezogen auf das Gewicht des ressultierenden Produkts, 0.01% bis 5% des Pfefferminzöls mit reduziertem Menthofuran-Gehalt einem Nahrungsmittel, Kaugummi, Konfekt, einem Pharmazeutikum, einem Getränk, Tabak oder in einem nichtrezeptpflichtigen Medikament beigibt.

8. Verfahren zum aromatisieren von Nahrungsmitteln, Kaugummi, Konfekt, einem Pharmazeutikum, einem Getränk, Tabak einem nichtrezeptpflichtigen Medikament, welches umfasst:

(1) das Herstellen eines Pfefferminzöls mit reduziertem Menthofuran-Gehalt mit einem Verfahren das umfasst:

(a) das Reagiern eines Ausgangs-Pfefferminzöls, bei einer Temperatur von 0°C bis 80°C, mit einer Menge von auf das Anfangsgewicht des Pfefferminzöls bezogen 0.5% bis 15% Maleinsäure-Anhydrid, um ein Menthofuran-Maleinsäure-Anhydrid-Addukt zu bilden, ohne die Aromaeigenschaften der verlieibenden Bestandteile des Pfefferminzöls zu beeinflussen;

(b) dass man der Reaktions-Mischung von Schritt (a) eine wässrige, basische Lösung zusetzt, um noch vorhandene Maleinsäure zu neutralisieren; und

(c) dass man das Produkt von Schritt (b) vakuum-oder wasserdampfdestilliert, um ein Pfefferminzöl zu gewinnen, das einen auf das Gewicht des Pfefferminzöls bezogenen Menthofuran-Gehalt von 0.8% bis 1.4% aufweist, und

(2) dass man dem Nahrungsmittel, Kaugummi, Konfekt, Pharmazeutikum, Getränk, Tabak oder nichtrezeptpflichtigen Medikament, eine wirksame Menge des reduziert menthofuranhaltigen Pfefferminzöls zufügt.

**Revendications**

1. Procédé de préparation d'essence de menthe poivrée à teneur réduite en menthofurane comprenant de:

(a) faire réagir à une température de 0°C à 80°C une essence de menthe poivrée de départ avec une quantité d'anhydride maléique dans la gamme de 0,5% à 15% en poids par rapport au poids de l'essence de menthe poivrée de départ pour former sélectivement un produit d'addition menthofurane-anhydride maléique sans affecter par ailleurs les propriétés aromatisantes des autres composantes de l'essence de menthe poivrée;

(b) mettre le mélange de réaction obtenu dans l'étape (a) en contact avec une solution aqueuse basique pour neutraliser tout acide maléique libre présent; et de

(c) soumettre le produit de l'étape (b) à une distillation sous vide ou un entraînement à la vapeur pour récupérer une essence de menthe poivrée ayant une teneur en menthofurane de 0,8% à 1,4% par rapport au poids de l'essence de menthe poivrée.

2. Procédé selon la revendication 1, où la teneur en menthofurane dans l'essence de menthe poivrée récupérée se situe dans la gamme de 0,8 à 1% en poids par rapport au poids de l'essence de menthe poivrée récupérée.

3. Procédé selon la revendication 1 ou 2, où la quantité d'anhydride maléique est la quantité stoechiométrique nécessaire pour réagir avec le menthofurane présent dans l'essence de menthe poivrée de départ.

4. Procédé selon la revendication 1 ou 2, où la quantité d'anhydride maléique est inférieure à la quantité stoechiométrique nécessaire pour réagir avec le menthofurane présent dans l'essence de menthe poivrée de départ.

5. Procédé selon l'une quelconque des revendications 1 à 4, où l'étape (b) comprend de mélanger le milieu de réaction avec une solution aqueuse basique pour former deux phases qui sont de préférence séparées, à savoir une phase organique comprenant l'essence de menthe poivrée et le produit d'addition menthofurane-anhydride maléique et une phase aqueuse comprenant une base libre, un sel de l'acide maléique et des impuretés hydrosolubles.

6. Procédé selon l'une quelconque des revendications qui précèdent, où le mélange de réaction comprenant le produit d'addition menthofurane-anhydride maléique est séparé en refroidissant le mélange de réaction en-dessous de sa température de réaction pour cristalliser le produit d'addition menthofurane-anhydride maléique.

7. Méthode de production d'une composition qui comprend de:

(1) préparer une essence de menthe poivrée à teneur réduite en menthofurane par un procédé comprenant de:

(a) faire réagir à une température de 0°C à 80°C une essence de menthe poivrée de départ avec une quantité d'anhydride maléique dans la gamme de 0,5% à 15% en poids par rapport au poids le l'essence de menthe poivrée de départ pour former sélectivement un produit d'addition menthofurane-anhydride maléique sans affecter par ailleurs les propriétés aromatisantes des autres composantes de l'essence de menthe poivrée;

(b) mettre le mélange de réaction obtenu dans l'étape (a) en contact avec une solution aqueuse basique pour neutraliser tout l'acide maléique libre présent; et de

(c) soumettre le produit de l'étape (b) à une distillation soud vide ou un entraînement à a vapeur pour récupérer une essence de menthe poivrée ayant une teneur en menthofurane de 0,8% à 1,4% par rapport au poids de l'essence de menthe poivrée; et de

(2) incorporer de 0,01% à 5% en poids par rapport au poids de la composition résultante de ladite essence de menthe poivrée à teneur réduite en menthofurane dans un article de confiserie, un produit pharmaceutique, une boisson, un tabec ou une autre spécialité.

8. Méthode d'aromatisation d'un aliment, d'un chewing-gum, d'un article de confiserie, d'un produit pharmaceutique, d'une boisson, d'un tabec ou d'une autre spécialité qui comprend de:

(1) préparer une essence de menthe poivrée à teneur réduite en menthofurane par un procédé comprenant de:

(a) faire réagir à une température de 0°C à 80°C une essence de menthe poivrée de départ avec une quantite d'anhydride maléique dans la gamme 0,5% à 15% en poids par rapport au poids de l'essence de menthe poivrée de départ pour former sélectivement un produit d'addition menthofurane-anhydride maléique sans affecter par ailleurs les propriétés aromatisantes de autres composantes de l'essence de menthe poivrée;

(b) mettre le mélange de réaction obtenu dans l'étape (a) en contact avec une solution aqueuse basique pour neutraliser tout acide maléique libre présent; et de

(c) soumettre le produit de l'étape (b) à une distillation sous vide ou à un entraînement à al vapeur pour récupérer une essence de menthe poivrée ayant une teneur en menthofurane de 0,8% à 1,4% par rapport au poids de l'essence de menthe poivrée; et de

(2) ajouter une quantité effective de ladite essence de menthe poivrée à tenuer réduite de menthofurane audit aliment, chewing-gum, article de confiserie, produit pharmaceutique, boisson, tabac ou autre spécialité.

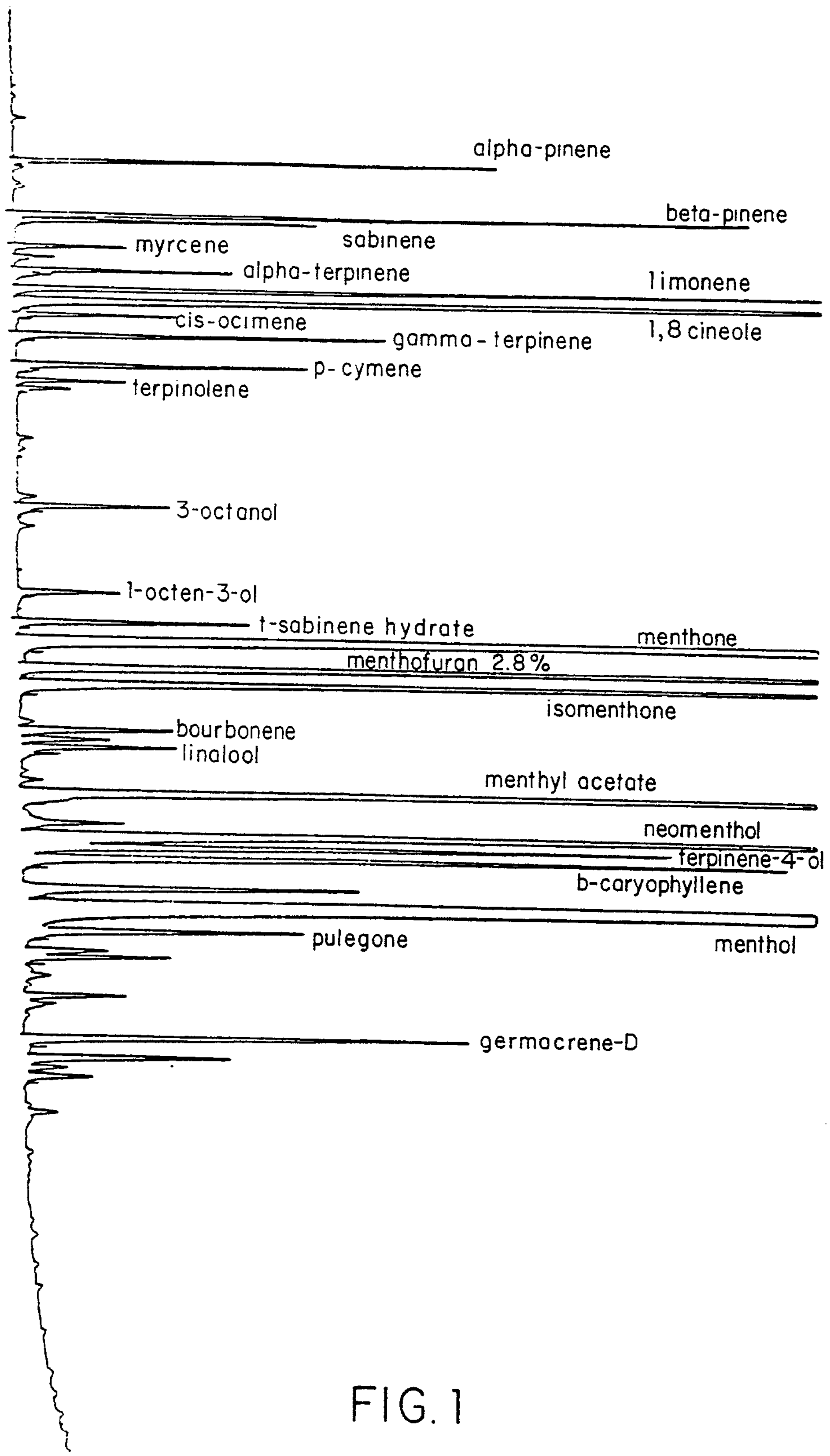
alpha-pinene
beta-pinene
myrcene
sabinene
alpha-terpinene
limonene
cis-ocimene
1,8 cineole
gamma-terpinene
p-cymene
terpinolene
3-octanol
1-octen-3-ol
t-sabinene hydrate
menthone
menthofuran 2.8%
isomenthone
bourbonene
linalool
menthyl acetate
neomenthol
terpinene-4-ol
b-caryophyllene
pulegone
menthol
germacrene-D

FIG. 1

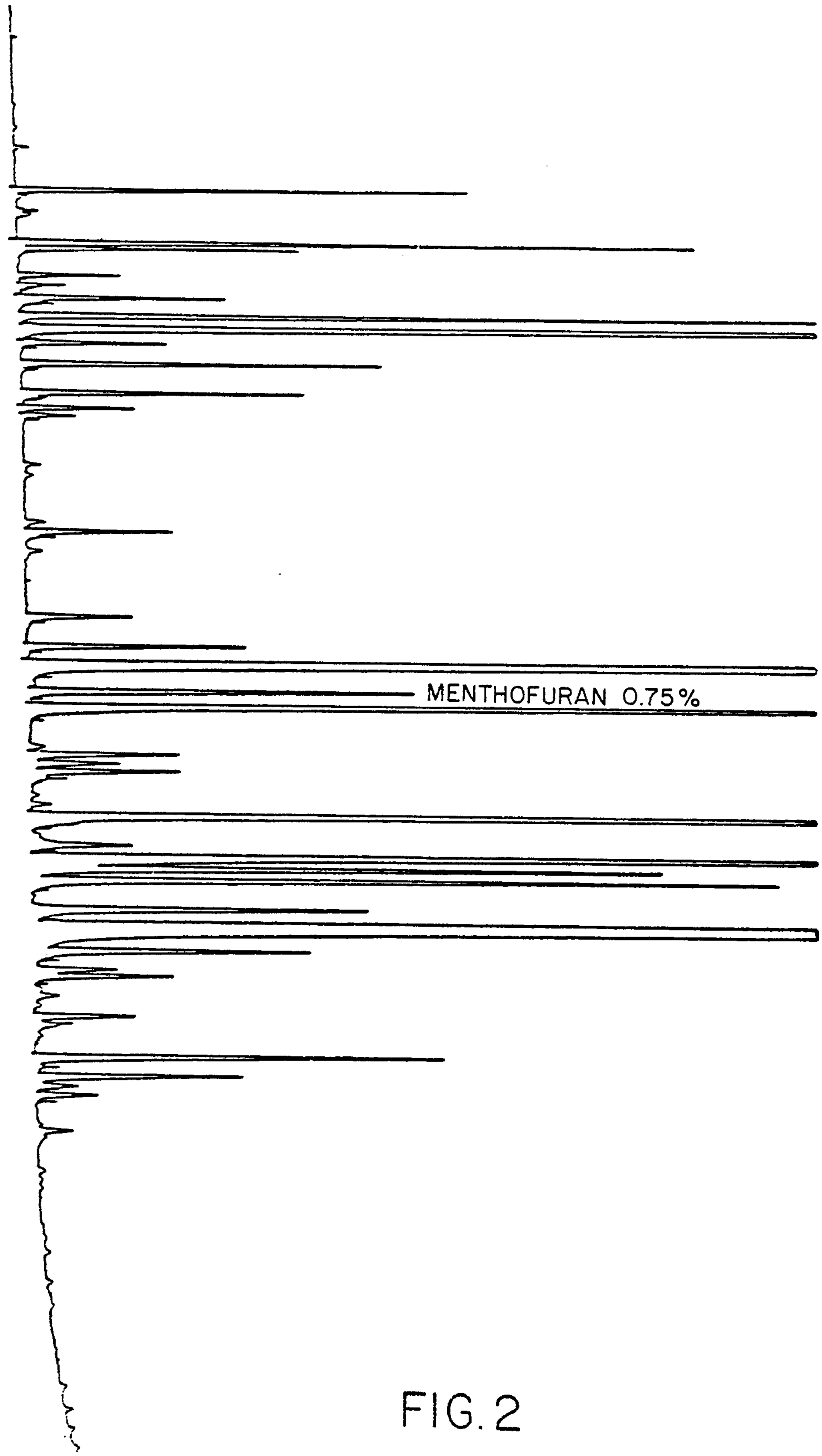


FIG. 2